# EUROPEAN PATENT SPECIFICATION

(11) **EP 2 060 243 B1**
(45) Date of publication and mention of the grant of the patent: **05.10.2011**
(21) Application number: 07120839.1
(22) Date of filing: 16.11.2007
(51) Int. Cl.: A61F 2/16, A61M 5/315, A61M 37/00

(54) **Insertion device for intraocular lens**
Einsetzvorrichtung für eine Intraokularlinse
Dispositif d'insertion pour lentille intraoculaire

(43) Date of publication of application: 20.05.2009
(73) Proprietor: STAAR Japan Inc., Urayasu-shi Chiba 279-0012 (JP)
(72) Inventor: Kobayashi, Kenichi, Ichikawa-shi Chiba (JP)
(74) Representative: TBK

(56) References cited:
- WO-A-2004/026377
- US-A- 4 402 308
- US-A- 5 222 972

## Description

### BACKGROUND OF THE INVENTION

The present invention relates to an insertion device for inserting into an eye an intraocular lens that is inserted instead of a crystalline lens after the crystalline lens is extracted because of cataract or inserted into an eye in order to cure abnormal refraction.

In current operations for cataract, the central portion of the anterior capsule of an eyeball is ablated, a clouded crystalline lens is removed by an ultrasonic suction apparatus, and then an artificial intraocular lens (hereinafter simply referred to as a lens) is placed in the position of the removed clouded crystalline lens. When placing the lens in the eyeball, an operation method for inserting the lens into the eyeball through a small incision by using the flexibility of the lens and thereby deforming the lens, e.g. folding the lens into a small shape is the mainstream.

In the case of an operation, an insertion device is frequently used which deforms a lens set in a main body of the device into a small shape while moving the lens in the main body of the device by a pushing shaft and pushes out the lens into the eye from a front end opening of an insertion cylinder (nozzle) inserted into the incision. This insertion device is used not only for the operation of cataract but also for a lens inserting operation for an eyesight correction medical treatment.

When the lens is inserted into an eye by using the insertion device, a viscoelastic material such as sodium hyaluronate is introduced into the main body of the insertion device as a lubricant so that the lens is smoothly moved and deformed in the insertion device (see Japanese Patent Laid-Open No. 2004-351196). Moreover, the viscoelastic material has a function of spreading the space of the anterior chamber of the eye into which the lens will be inserted by being introduced into the eye through the insertion cylinder.

Further, it has been recently required to use inexpensive physiologic saline in place of the viscoelastic material.

In the case of a conventional operation, a liquid is introduced into the insertion device immediately before the operation, which takes time and places a heavy burden on an operator or an assistant.
Document US 5, 222, 972 discloses a forceps comprising first and second elongated elements having proximal and distal end portions. The proximal end portions are joined together so that the distal end portions are movable toward and away from each other. The distal end portion of the first elongated element includes a platform having a substantially flat surface sized and adapted to carry a foldable intraocular lens in an unfolded state. The distal end portion of the second elongated element includes an elongated rod which is narrower than the substantially flat surface of the platform.

### SUMMARY OF THE INVENTION

The present invention provides an insertion device for an intraocular lens that can facilitate introduction of liquid into the insertion device.

The present invention in its first aspect provides an insertion device for an intraocular lens as specified in claims 1 to 3.

The present invention in its second aspect provides a manufacturing method for the intraocular-lens-preloaded type insertion device as specified in claim 4.

Other aspects of the present invention will be apparent from the embodiments described below with reference to the drawings.

### BRIEF DESCRIPTION OF THE DRAWINGS

FIG. 1 is a sectional view of an insertion device that is an embodiment of the present invention, which has a pushing shaft of a two-shaft configuration.

FIG. 2 is a perspective view for illustrating a liquid introduction method into the insertion device shown in FIG. 1.

FIG. 3 is a sectional view for illustrating an operation of the insertion device shown in FIG. 1.

FIG. 4 is a figure for illustrating an operation method of the insertion device shown in FIG. 1 in an operation of the eye.

FIG. 5 is a sectional view of the pushing shaft in FIG. 1.

FIG. 6 is a sectional view of a modified example of the pushing shaft of the two-shaft configuration.

FIG. 7 is a sectional view of an insertion device having a pushing shaft of a one-shaft configuration.

FIG. 8 is a figure for illustrating an operation method of the insertion device shown in FIG. 7 in an operation of the eye.

FIG. 9 is a figure for illustrating an operation method of the insertion device shown in FIG. 7 in an operation of the eye.

### DETAILED DESCRIPTION OF THE PREFERRED EMBODIMENTS

Exemplary embodiments of the present invention will be described below with reference to the accompanied drawings.

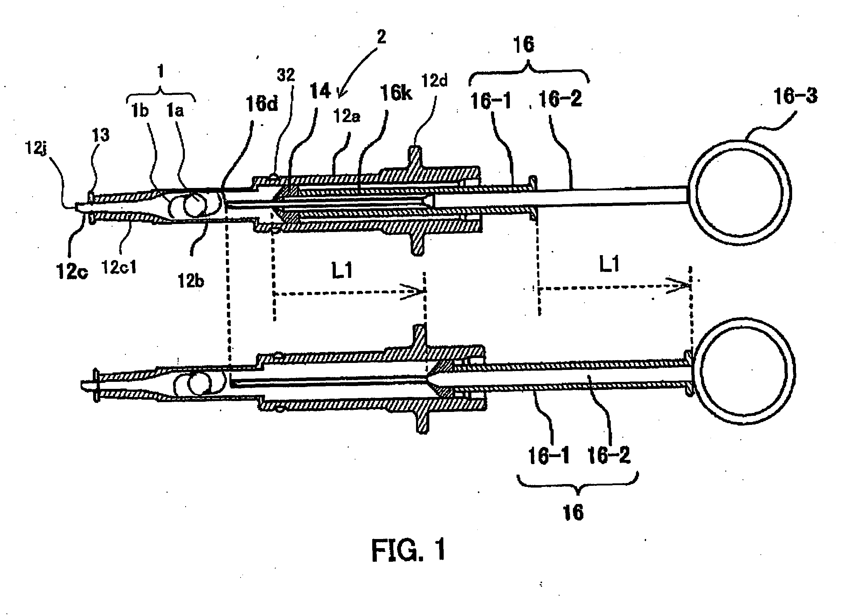

FIG. 1 shows an insertion device for an intraocular lens (hereinafter simply referred to as a lens) that is an embodiment of the present invention. In the description below, a nozzle side is referred to as a front side, and a side opposite to the nozzle side is referred to as a rear side. A direction extending toward the front side and the rear side is referred to as an axial direction.

An insertion device 2 is basically constituted by a main body with a nozzle (hereinafter simply referred to as a main body) 12, and a pushing shaft 16. The main body 12 includes an outer cylindrical portion 12a as a hand-held portion having an outer diameter suitable for holding the insertion device 2 by hand, a lens housing portion 12b that is provided on the side closer to the front end than the outer cylindrical portion 12a and houses a lens 1, and a nozzle portion 12c as an insertion cylindrical portion provided on the side closer to the front end than the lens housing portion 12b. The main body 12 is an integrally formed component (member). On the rear of the outer cylindrical portion 12a, a flange portion 12d is formed as a portion supported by hand when pushing the pushing shaft 16.

The main body 12 has a hollow shape, and the pushing shaft 16 is inserted through a rear end opening thereof.

The nozzle portion 12c has decreasing inner and outer diameters toward the front end, and a portion from a front end opening 12j to a predetermined length is formed to be the thinnest portion in the nozzle portion 12c as a portion to be inserted into an eye through an incision formed in an eyeball. On an outer periphery of the rear end of the insertion portion, a cover ring (an O-ring) 13 made of an elastic member such as rubber is mounted. On the rear side of the cover ring 13 in the nozzle portion 12c, a step 12cl is formed having a larger outer diameter than the insertion portion for preventing rearward movement of the cover ring 13.

The lens housing portion 12b has an inner surface shape capable of stably holding the lens 1 inserted from the rear end opening of the main body 12, though not shown in the figure.

The lens 1 has a circular shape in top view, and includes an optical portion 1a having the function of a lens and support portions 1b extending from the front end and the rear end of the optical portion 1a. The support portion 1b is a wire-like portion that elastically supports the optical portion 1a in the eye after the lens 1 is inserted into the eye.

The lens housing portion 12b supports the lens 1 in a state in which a stress by the weight of the lens 1 or an external force is not substantially applied. The state in which the stress is not substantially applied denotes a state in which no stress is applied to the optical portion 1a at all as well as a state in which a minute stress is applied so that a deformation influencing the optical function of the optical portion 1a after insertion of the lens 1 into the eye does not occur even if the lens 1 is held and stored for a long time. In other words, the state denotes a state in which a stress or a deformation influencing the optical function of the optical portion la does not occur.

The main body 12 basically has no opening except the rear end opening of the outer cylindrical portion 12a and the front end opening 12j of the nozzle portion 12c. However, a small hole is formed in a peripheral wall near the front end of the outer cylindrical portion 12a. This small hole is naturally formed for placing a member that supports a die for forming the inner surface of the main body 12 in production of the main body 12, that is, when the main body 12 is integrally formed of resin. The small hole is covered by mounting an O-ring 32 made of an elastic member such as rubber on the peripheral wall of the outer cylindrical portion 12a. This achieves the main body 12 having no opening other than the rear end opening of the outer cylindrical portion 12a and the front end opening 12j of the nozzle portion 12c. If not covering the small hole does not directly influence the flow of the liquid, covering of the small hole is not necessarily required.

The rear end opening of the outer cylindrical portion 12a is covered with a seal cap 14 provided on the pushing shaft 16 without a gap, and the front end opening 12j is covered with a cap (not shown) without a gap, thereby allowing a sealed space to be formed in the main body 12 that can house a liquid such as a viscoelastic material or physiologic saline without leakage, and store the liquid with the lens 1.

The O-ring 32 is provided on a position often touched by hand of an operator holding the insertion device 2. Thus, the O-ring 32 has the function of covering the above-described small hole as well as the function of preventing slip of the hand holding the insertion device 2.

The pushing shaft portion 16c has a small outer diameter that can pass through an inner passage of the nozzle portion 12c, and has, at the front end thereof, a lens grip portion (lens contact portion) 16d vertically bifurcated.

Into the insertion device 2 assembled as described above, a liquid is introduced such as a viscoelastic material such as sodium hyaluronate or physiologic saline (including one in which drug is dissolved) as described later. A liquid introduction method will be described below. A liquid to be introduced includes hydrophilic (water-soluble) polymer liquid besides sodium hyaluronate or physiologic saline. For example, synthetic polymer includes polyethylene glycol (PEG), polypropylene glycol (PPG), sodium polyacrylate (PAA), polyacrylamide (PAAm), sodium polystyrene sulfonate (PSSNa), polyvinylpyrrolidone (PVP), polyvinyl alcohol (PVA), polyethyleneimine (PEI), carboxymethylcellulose (CMC), dextran sodium sulfate, hydroxyethyl starch (HEPES), and polyphosphoric acid. Natural polymer includes hyaluronate and/or sodium hyaluronate (HA), sodium alginate, dextran, dextrin, heparin, chitosan, and sodium chondroitin sulfate as polysaccharide, and polypeptide and polynucleic acid as other than polysaccharide.

Among them, polysaccharide is preferably used in view of biocompatibility or the diversity of molecular weight obtained.

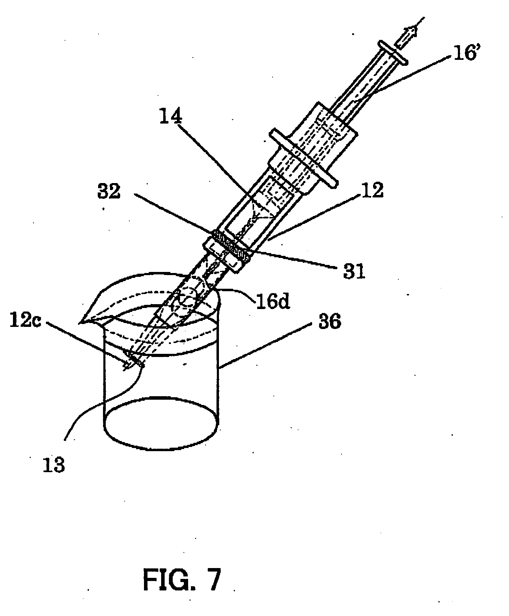

FIG. 7 shows a liquid introduction method in a general insertion device. Portions and components having functions identical to those in the insertion device shown in FIG. 1 are designated with the same reference numerals as those in FIG. 1.

The liquid is placed in a container 36 such as a sterile beaker, and a pushing shaft 16' is drawn rearward in a state in which the nozzle portion 12c is inserted in the liquid. The main body 12 is sealed except the front end opening 12j, thereby allowing the liquid to be introduced (filled) into the main body 12 similarity to a syringe.

However, when the pushing shaft 16' is a single-shaft type that is integrally formed and has the lens grip portion 16d, it is necessary that the pushing shaft 16' be drawn rearward from a state in which the lens grip portion 16d is located slightly rearward of the optical portion 1a of the lens 1 in the lens housing portion 12b. Therefore, the length by which the pushing shaft 16' can be drawn rearward is short, thereby making it difficult to introduce a sufficient amount of the liquid into the main body 12.

In contrast, in this embodiment, in order to introduce a sufficient amount of the liquid into the main body 12 by a similar operation to that for the syringe, the pushing shaft 16 has a two-shaft configuration as shown FIG. 1.

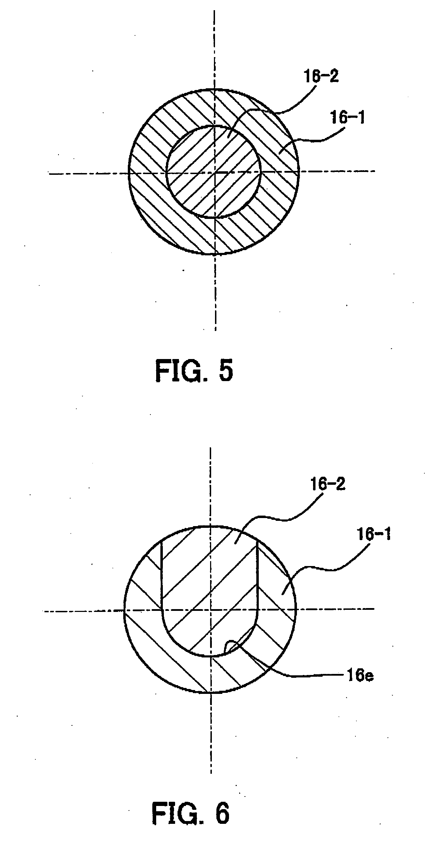

Specifically, a through hole (opening) 16k is formed in an outer shaft member (second member) 16-1 with the seal cap 14 mounted at the tip thereof, the through hole 16k extending in the axial direction of the outer shaft member 16-1. Then, a pushing member (first member) 16-2 having the lens grip portion 16d at the tip thereof is inserted into the through hole 16k to be assembled to the outer shaft member 16-1. The outer shaft member 16-1 and the pushing member 16-2 are concentrically assembled as shown in FIG. 5. The pushing member 16-2 is held so as to be movable in the axial direction by the outer shaft member 16-1. The outer shaft member 16-1 is a member having no function of pushing the lens 1, which is different from the pushing member 16-2 having the lens grip portion 16d, that is, a function of pushing the lens 1.

The inner surface of the outer shaft member 16-1 is in close contact with the outer surface of the pushing member 16-2, which prevents leakage of the liquid through therebetween. The pushing member 16-2 and the outer shaft member 16-1 are movable independently from each other in the axial direction with respect to the main body 12. In other words, it is possible to move with respect to the main body 12 only the pushing member 16-2, only the outer shaft member 16-1, or both the members 16-1 and 16-2.

The upper figure of FIG. 1 shows a state in which the pushing member 16-2 is set such that the lens grip portion 16d is located slightly rearward of the lens 1 and the outer shaft member 16-1 is inserted up to the vicinity of the mounted position of the O-ring 32 in the main body 12. The lower figure of FIG. 1 shows a state in which the pushing member 16-2 is set at the same position as that shown in the upper figure of FIG. 1 and the outer shaft member 16-1 is moved rearward from the position shown in the upper figure.

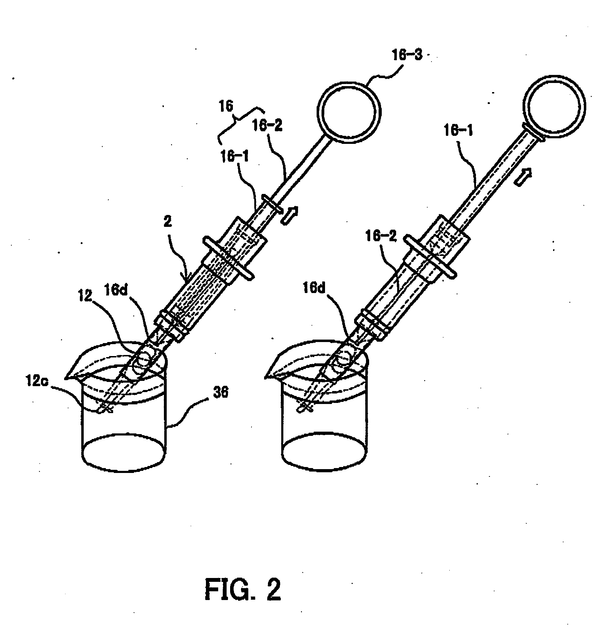

When the liquid is introduced into the insertion device 2 having the pushing shaft 16 of such a two-shaft configuration, as shown in FIG. 2, the nozzle portion 12c of the insertion device 2 being in the state shown in the upper figure of FIG. 1 is inserted in the liquid in the container (beaker) 36 and then the outer shaft member 16-1 is drawn rearward with the pushing member 16-2 fixed. This enables the seal cap 14, as shown in the lower figure of FIG. 1, to move rearward with the lens grip portion 16d of the pushing member 16-2 located slightly rearward of the lens 1. This allows the liquid to be introduced (filled) into the main body 12 similarly to the syringe.

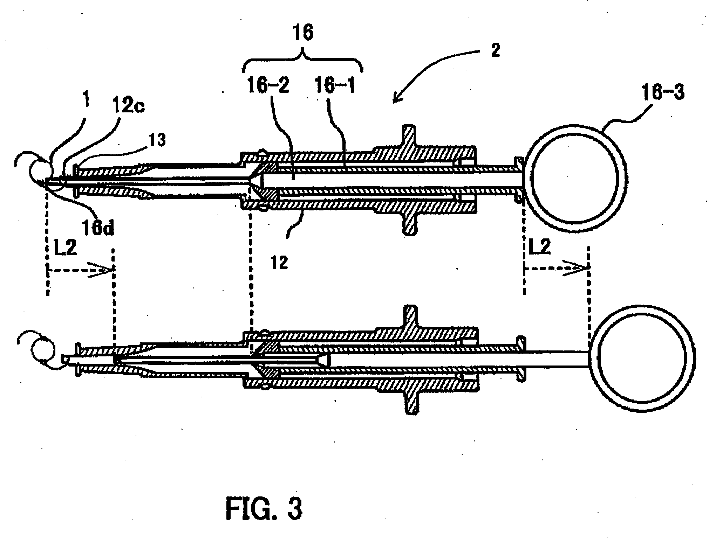

In an operation (surgery), an operation ring (interlocking portion) 16-3 provided at the rear end of the pushing member 16-2 is held by a hand to push the pushing member 16-2 frontward from the state shown in the lower figure of FIG. 1 (the right figure of FIG. 2). The operation ring 16-3 is thereby brought into contact with the rear end of the outer shaft member 16-1, as shown in the upper figure of FIG. 3, to move the outer shaft member 16-1 frontward together with the pushing member 16-2. Thereby, the lens 1 is pushed out through the nozzle portion 12c by the lens grip portion 16d of the pushing member 16-2 to be inserted into the eye, and the liquid filled in the main body 12 is flowed into the eye by the forward movement of the seal cap 14.

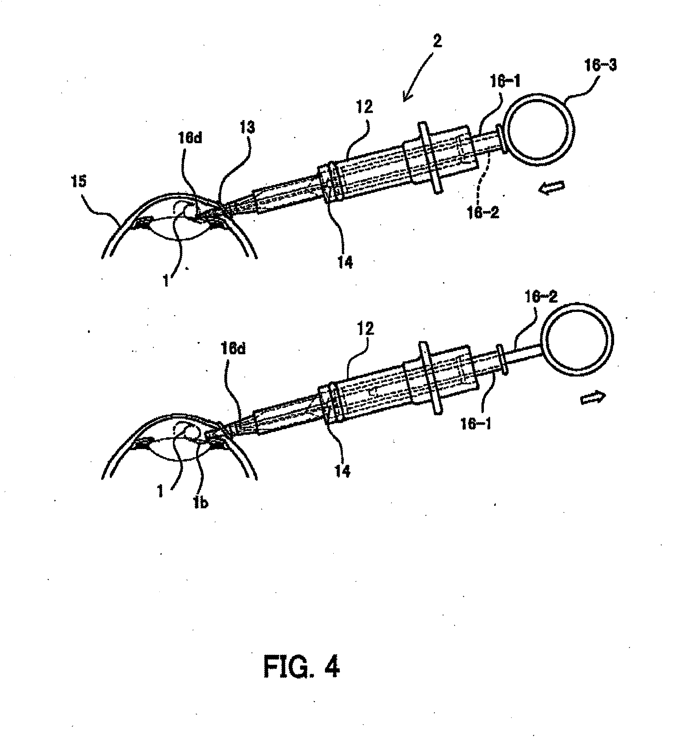

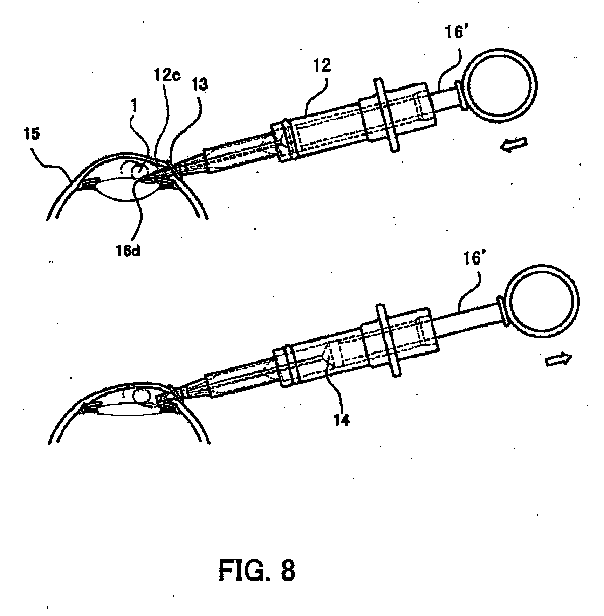

FIG. 4 and FIG. 8 show an operation using the pushing shaft 16 of the two-shaft configuration and an operation using the pushing shaft 16' of the one-shaft configuration, respectively.

In the operation using the pushing shaft 16' of the one-shaft configuration shown in FIG.8, when the pushing shaft 16' is drawn rearward as shown in the lower figure after the nozzle portion 12c is inserted into an incision on an eyeball 15 and then the lens 1 is pushed into the eye as shown in the upper figure, the seal cap 14 mounted to the pushing shaft 16' is moved rearward. Thereby, aqueous fluid in the eye is flowed back to the main body 12.

In contrast, in the operation using the pushing shaft 16 of the two-shaft configuration shown in FIG. 4, even when the pushing member 16-2 is drawn rearward as shown in the lower figure from the state shown in the upper figure, the outer shaft member 16-1 on which the seal cap 14 is mounted is not moved. In other words, only the lens grip portion 16d of the pushing member 16-2 is moved rearward. Thereby, the backflow of the aqueous fluid in the eye to the main body 12 is significantly reduced.

When using the insertion devices shown in FIG. 8 and FIG. 4, the cover ring 13 mounted on the nozzle portion 12c is brought into close contact with an area around the incision on the eyeball 15 to limit outflow of the liquid through a gap between the incision and the nozzle portion 12c. In such an operation, the use of the pushing shaft 16' of the one-shaft configuration particularly easily causes the above-described backflow of the aqueous fluid. However, the use of the pushing shaft 16 of the two-shaft configuration can effectively reduce the backflow of the aqueous fluid.

Further, as shown in the lower figure of FIG. 4, after the lens 1 is inserted into the eye, the pushing member 16-2 can be operated to easily perform lens repositioning (e.g., positional adjustment of the support portion 1b) in the eye.

Reference character L1 in FIG. 1 denotes the movement amount of the seal cap 14 in the insertion device 2 having the pushing shaft 16 of the two-shaft configuration when the outer shaft member 16-1 is drawn rearward for the introduction of the liquid into the main body 12. The rearward movement of the outer shaft member 16-1 by L1 moves the seal cap 14 rearward by the same amount without changing the position of the lens grip portion 16d of the pushing member 16-2.

Reference character L2 in FIG. 3 denotes the movement amount of the lens grip portion 16d in the insertion device 2 having the pushing shaft 16 of the two-shaft configuration when the lens repositioning is performed. The rearward movement of the pushing member 16-2 by L2 moves the lens grip portion 16d rearward from the inside of the eye into the main body 12 by the same amount without changing the position of the seal cap 14. This enables the lens repositioning with little backflow of the aqueous fluid from the eye into the main body 12.

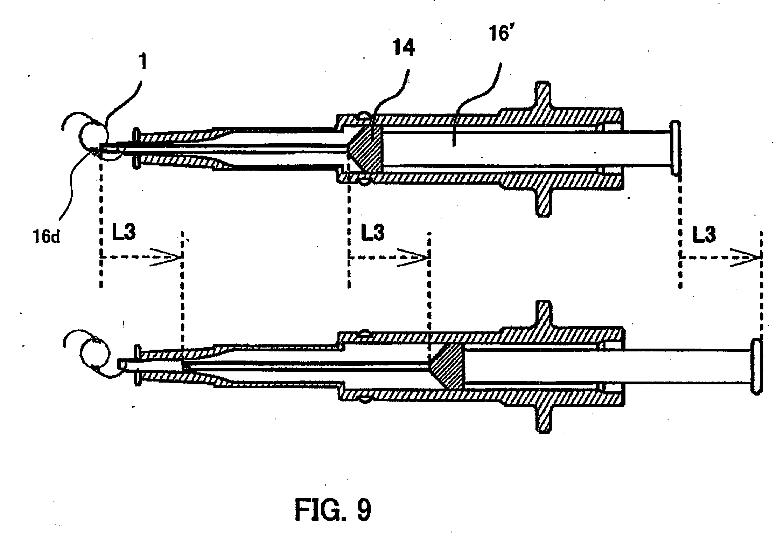

On the other hand, reference character L3 in FIG. 9 denotes the movement amount of the lens grip portion 16d and the seal cap 14 in the insertion device having the pushing shaft 16' of the one-shaft configuration when the lens repositioning is performed. The rearward movement of the pushing shaft 16' by L3 moves not only the lens grip portion 16d but also the seal cap 14 rearward by the same amount. This causes the backflow of the aqueous fluid from the eye into the main body 12.

The description was made of the pushing shaft 16 having the concentric two-shaft configuration in which the pushing member (first member) 16-2 is inserted into the through hole 16k formed in the outer shaft member (second member) 16-1, which is shown in FIG. 5. However, other two-shaft configurations may be employed as long as the first and second members can move independently from each other in the axial direction For example, as shown in FIG. 6, a U-shaped groove 16e extending in the axial direction is formed in a second member 16-1, and a first member 16-2 is put in (held by) the groove 16e movably in the axial direction. This two-shaft configuration can prevent the rotation of the first member 16-2 with respect to the second member 16-1 and facilitate forming of the pushing shaft 16 of the two-shaft configuration.

Alternatively, the first and second members may respectively have a lens contact portion that makes contact with and pushes the lens 1.

The assembling procedure (manufacturing method) of the insertion device (intraocular-lens-preloaded type insertion device) 2 configured as described above will be simply described. First, the main body 12 before the pushing shaft 16 is inserted thereinto is prepared. The lens 1 is inserted into the main body 12 from the rear end opening of the main body 12, and then the lens 1 is placed in the lens housing portion 12b.

Next, the pushing shaft 16 assembled by inserting the pushing member 16-2 into the through hole 16k formed in the outer shaft member 16-1 is inserted into the main body 12 from its rear end opening.

Then, the O-ring 32 and cover ring 13 are mounted on the main body 12. Further, the liquid is introduced into the main body 12 by the above-described method, and then a cap (not shown) is attached to the nozzle portion 12c to seal its front end opening 12j. Thus, the manufacturing of the intraocular-lens-preloaded type insertion device 2 in which the lens 1 is preliminarily loaded in the lens housing portion 12b is completed.

As described above, the insertion device has the pushing shaft of the two-shaft configuration in which the first and second members can move independently from each other in the axial direction with respect to the main body, so that a sufficient amount of the liquid can be introduced into the main body by moving only the second member, similarly to the syringe. Further, moving the first and second members can insert the lens and liquid into the eye. Moreover, moving only the first member after the insertion of the lens into the eye enables the lens repositioning in the eye while preventing the backflow of the aqueous fluid.

In the embodiment, a so-called preload type insertion device (intraocular-lens-preloaded type insertion device) has been described in which the lens 1 is previously set in the lens housing portion 12b before factory shipment (before delivery to a hospital). However, alternative embodiments of the present invention may include other types of insertion devices. For example, an insertion device in which it is stored separately from a lens and the lens 1 is set immediately before an operation can be used.

Furthermore, the present invention is not limited to this embodiment and various variations and modifications may be made without departing from the scope of the present invention as claimed.
An insertion device (2) for an intraocular lens (1) includes a main body (12) in which the lens is placed, and a pushing shaft (16) that pushes out the lens from the main body into an eye (15). The pushing shaft is constituted by a first member (16-2) and a second member (16-1) that are movable independently from each other in an axial direction of the device witch respect to the main body. The insertion device can facilitate introduction of liquid thereinto.

## Claims

1. An insertion device (2) for an intraocular lens (1) comprising:
a main body (12) in which the lens is placed; and
a pushing shaft (16) that pushes out the lens from the main body into an eye (15),
**characterized in that** the pushing shaft is constituted by a first member (16-2) and a second member (16-1) that are movable independently from each other in an axial direction of the device with respect to the main body.

2. An insertion device according to claim 1, wherein the first member (16-2) makes contact with and pushes the lens (1), and the second member (16-1) does not make contact with the lens.

3. An insertion device (2) according to claim 1 or 2, wherein the device is an intraocular-lens-preloaded type insertion device including the intraocular lens (1) placed in a lens housing portion (12b) that is formed in the main body (12).

4. A manufacturing method for an intraocular-lens-preloaded type insertion device, **characterized by** comprising the steps of:
preparing an insertion device (2) comprising:
a main body (12) in which an intraocular lens (1) is placed; and a pushing shaft (16) that pushes out the lens from the main body into an eye (15), the pushing shaft being constituted by a first member (16-2) and a second member (16-1) that are movable independently from each other in an axial direction of the device with respect to the main body, and
placing the lens to a lens housing portion (12b) that is formed in the main body.

## Patentansprüche

1. Einsetzvorrichtung (2) für eine Intraokularlinse (1), die Folgendes aufweist:
einen Hauptkörper (12), in dem die Linse platziert ist; und
eine Drückspindel (16), die die Linse aus dem Hauptkörper in ein Auge (15) drückt,
**dadurch gekennzeichnet, dass**
die Drückspindel durch ein erstes Bauteil (16-2) und ein zweites Bauteil (16-1) gebildet ist, die unabhängig voneinander in einer axialen Richtung der Vorrichtung hinsichtlich des Hauptkörpers bewegbar sind.

2. Einsetzvorrichtung (2) nach Anspruch 1, wobei das erste Bauteil (16-2) einen Kontakt mit der Linse (1) herstellt und diese drückt und das zweite Bauteil (16-1) keinen Kontakt mit der Linse herstellt.

3. Einsetzvorrichtung (2) nach Anspruch 1 oder 2,wobei die Vorrichtung eine mit einer Intraokularlinse vorab beladene Einsetzvorrichtung ist, die die Intraokularlinse (1) aufweist, die in einem Linsenunterbringungsabschnitt (12b) platziert ist, der in dem Hauptkörper (12) ausgebildet ist.

4. Herstellungsverfahren für eine mit einer Intraokularlinse vorab beladene Einsetzvorrichtung, **gekennzeichnet durch** die folgenden Schritte:
Bereitstellen einer Einsetzvorrichtung (2), die Folgendes aufweist:
einen Hauptkörper (12), in dem eine Intraokularlinse (1) platziert ist; und eine Drückspindel (16), die die Linse aus dem Hauptkörper in ein Auge (15) herausdrückt, wobei die Drückspindel **durch** ein erstes Bauteil (16-2) und ein zweites Bauteil (16-1) gebildet ist, die unabhängig voneinander in einer axialen Richtung der Vorrichtung hinsichtlich des Hauptkörpers bewegbar sind, und
Platzieren der Linse an einem Linsenunterbringungsabschnitt (12b), der in dem Hauptkörper ausgebildet ist.

## Revendications

1. Dispositif d'insertion (2) pour une lentille intraoculaire (1) comprenant :
un corps principal (12) dans lequel la lentille est placée ; et
un arbre de poussée (16) qui pousse la lentille hors du corps principal pour qu'elle arrive dans un oeil (15),
**caractérisé en ce que** l'arbre de poussée est constitué par un premier élément (16-2) et un deuxième élément (16-1) qui sont mobiles indépendamment l'un de l'autre dans une direction axiale du dispositif par rapport au corps principal.

2. Dispositif d'insertion selon la revendication 1, dans lequel le premier élément (16-2) établit un contact avec la lentille (1) et la pousse, et le deuxième élément (16-1) n'établit pas de contact avec la lentille.

3. Dispositif d'insertion (2) selon la revendication 1 ou 2, dans lequel le dispositif est un dispositif d'insertion de type pré-chargé de lentille intraoculaire comportant la lentille intraoculaire (1) placée dans une partie (12b) de réception de lentille qui est formée dans le corps principal (12).

4. Procédé de fabrication pour un dispositif d'insertion de type pré-chargé de lentille intraoculaire, **caractérisé par** le fait de comprendre les étapes qui consistent :
à préparer un dispositif d'insertion (2) comprenant :
un corps principal (12) dans lequel une lentille intraoculaire (1) est placée ; et un arbre de poussée (16) qui pousse la lentille hors du corps principal pour qu'elle arrive dans un oeil (15), l'arbre de poussée étant constitué par un premier élément (16-2) et un deuxième élément (16-1) qui sont mobiles indépendamment l'un de l'autre dans une direction axiale du dispositif par rapport au corps principal, et
à placer la lentille dans une partie (12b) de réception de lentille qui est formée dans le corps principal.
